# EUROPÄISCHE PATENTSCHRIFT

(11) **EP 1 458 716 B1**
(45) Veröffentlichungstag und Bekanntmachung des Hinweises auf die Patenterteilung: **27.09.2006**
(21) Anmeldenummer: 02787913.9
(22) Anmeldetag: 06.12.2002
(51) Int. Cl.: C07D 453/02, A61K 31/435, A61P 25/28

(54) **BICYCLISCHE N-ARYLAMIDE**
BICYCLIC N-ARYLAMIDES
N-ARYLAMIDES BICYCLIQUES

(30) Priorität: 19.12.2001 DE 10162375
(43) Veröffentlichungstag der Anmeldung: 22.09.2004
(73) Patentinhaber: Bayer HealthCare AG, 51368 Leverkusen (DE)
(72) Erfinder: LUITHLE, Joachim, 42489 Wülfrath (DE); BÖSS, Frank-Gerhard, Berkshire SL5 7DF (GB); ERB, Christina, 65830 Kriftel (DE); FLESSNER, Timo, 42113 Wuppertal (DE); HENDRIX, Martin, 51519 Odenthal (DE); VAN KAMPEN, Marja, 63263 Neu-Isenberg (DE); METHFESSEL, Christoph, 42327 Wuppertal (DE)
(86) Internationale Anmeldenummer: PCT/EP2002/013835
(87) Internationale Veröffentlichungsnummer: WO 2003/051874

(56) Entgegenhaltungen:
- EP-A- 0 190 920
- WO-A-01/60821
- DATABASE CA [Online] CHEMICAL ABSTRACTS SERVICE, COLUMBUS, OHIO, US; BINIECKI, STANISLAW ET AL: "Synthesis of quinaldinylamides and lepidinylamides of quinuclidine-2-carboxylic acid" retrieved from STN Database accession no. 88:37590 XP002234092 -& ACTA POLONIAE PHARMACEUTICA ( 1977 ), 34(2), 125-31 , XP008014839

## Beschreibung

Die Erfindung betrifft neue bicyclische N-Arylamide, Verfahren zu ihrer Herstellung sowie ihre Verwendung zur Herstellung von Arzneimitteln zur Behandlung und/oder Prophylaxe von Krankheiten und zur Verbesserung der Wahrnehmung, Konzentrationsleistung, Lernleistung und/oder Gedächtnisleistung.

Nikotinische Acetylcholin-Rezeptoren (nAChR) bilden eine große Familie von Ionenkanälen, die durch den körpereigenen Botenstoff Acetylcholin aktiviert werden (Galzi and Changeux, *Neuropharmacol.* **1995,** *34*, 563-582). Ein funktioneller nAChR besteht aus fünf Untereinheiten, die unterschiedlich (bestimmte Kombinationen von α1-9 und β1-4,γ,δ,ε-Untereinheiten) oder identisch, (α7-9) sein können. Dies führt zur Bildung einer Vielfalt von Subtypen, die eine unterschiedliche Verteilung in der Muskulatur, dem Nervensystem und anderen Organen zeigen (McGehee and Role, *Annu. Rev. Physiol.,* **1995,** *57,* 521-546). Aktivierung von nAChR führt zum Einstrom von Kationen in die Zelle und zur Stimulation von Nerven- oder Muskelzellen. Selektive Aktivierung einzelner nAChR-Subtypen beschränkt diese Stimulation auf die Zelltypen, die den entsprechenden Subtyp besitzen und kann so unerwünschte Nebeneffekte wie z.B. die Stimulierung von nAChR in der Muskulatur vermeiden. Klinische Experimente mit Nikotin und Experimente in verschiedenen Tiermodellen weisen auf eine Rolle von zentralen nikotinischen Acetylcholin-Rezeptoren bei Lern- und Gedächtnisvorgängen hin (z.B. Rezvani and Levin, *Biol. Psychiatry* **2001,** *49,* 258-267). Nikotinische Acetylcholinrezeptoren des alpha7-Subtyps (α7-nAChR) haben eine besonders hohe Konzentration in für Lernen und Gedächtnis wichtigen Hirnregionen, wie dem Hippocampus und dem cerebralen Cortex (Séguéla et al., *J. Neurosci.* **1993**, *13*, 596-604). Der α7-nAChR besitzt eine besonders hohe Durchlässigkeit für Calcium-Ionen, erhöht glutamaterge Neurotransmission, beeinflusst das Wachstum von Neuriten und moduliert auf diese Weise die neuronale Plastizität (Broide and Leslie, *Mol. Neurobiol.* **1999***, 20,* 1-16).

Bestimmte Chinuclidincarbonsäureanilide sind als Antiarrhythmika und Lokalanästhetika beschrieben (vgl. beispielsweise FR 1.566.045, GB 1 578 421 und Oppenheimer et al. *Life Sci.* **1991,** *48,* 977-985).

Die WO 01/60821 offenbart Biarylcarbonsäureamide mit Affinität zum α7-nAChR zur Behandlung von Lern- und Wahrnehmungsstörungen.

Die vorliegende Erfindung betrifft Verbindungen der allgemeinen Formel (I), 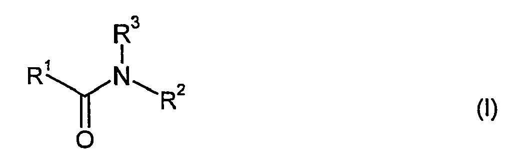 in welcher
- R¹: für 1-Aza-bicyclo[3.2.1]octyl, 1-Aza-bicyclo[3.3.1]nonyl oder 1-Aza-bicyclo[2.2.2]oct-3-yl steht
wobei der Bicycloalkylrest gegebenenfalls durch (C₁-C₆)-Alkyl substituiert ist,
- R²: für 8- bis 10-gliedriges Heteroaryl, Naphthyl oder Azulenyl steht, wobei die Ringe gegebenenfalls durch Reste ausgewählt aus der Gruppe Wasserstoff, Halogen, Formyl, Carbamoyl, Cyano, Trifluormethyl, Trifluormethoxy, Nitro, (C₁-C₆)-Alkyl, (C₁-C₆)-Alkoxy, (C₁-C₆)-Alkylthio substituiert sind,
und
- R³: für Wasserstoff oder (C₁-C₆)-Alkyl steht.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomere und Diastereomere lassen sich in bekannter Weise in die stereoisomer einheitlichen Bestandteile trennen.

Die erfindungsgemäßen Verbindungen können auch in Form ihrer Salze, Solvate oder Solvate der Salze vorliegen.

Als Salze sind im Rahmen der Erfindung physiologisch unbedenkliche Salze der erfmdungsgemäßen Verbindungen bevorzugt.

Physiologisch unbedenkliche Salze der erfindungsgemäßen Verbindungen können Säureadditionssalze der Verbindungen mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ehansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können aber auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabiethylamin, 1-Ephenamin oder Methyl-piperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten im Allgemeinen die folgende Bedeutung:
(C₁-C₆)-und(C₁-C₄)-Alkoxy steht für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4, besonders bevorzugt mit 1 bis 3 Kohlenstoffatomen. Beispielsweise und vorzugsweise seien genannt: Methoxy, Ethoxy, n-Propoxy, Isopropoxy, tert.Butoxy, n-Pentoxy und n-Hexoxy.
(C₁-C₆)- und (C₁-C₄)-Alkyl stehen für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4, besonders bevorzugt mit 1 bis 3 Kohlenstoffatomen. Beispielsweise und vorzugsweise seien genannt: Methyl, Ethyl, n-Propyl, Isopropyl, tert.Butyl, n-Pentyl und n-Hexyl.
(C₁-C₆)-Alkylthio steht für einen geradkettigen oder verzweigten Alkylthiorest mit 1 bis 6 Kohlenstoffatomen. Bevorzugt ist ein geradkettiger oder verzweigter Alkylthiorest mit 1 bis 4, besonders bevorzugt mit 1 bis 3 Kohlenstoffatomen. Beispielsweise und vorzugsweise seien genannt: Methylthio, Ethylthio, n-Propylthio, Isopropylthio, tert.Butylthio, n-Pentylthio und n-Hexylthio.
Halogen steht für Fluor, Chlor, Brom und Jod. Bevorzugt sind Fluor, Chlor und Brom. Besonders bevorzugt sind Fluor und Chlor.
8- bis 10-gliedriges Heteroaryl steht für einen aromatischen, bicyclischen Rest mit 8 bis 10, vorzugsweise 9 bis 10 Ringatomen und bis zu 5, vorzugsweise bis zu 4 Heteroatomen aus der Reihe S, O und/oder N. Der Heteroarylrest kann über ein Kohlenstoff- oder Heteroatom gebunden sein. Beispielsweise und vorzugsweise seien genannt: Indolyl, Indazolyl, Benzofuranyl, Benzothiophenyl, Chinolinyl, Isochinolinyl.

Wenn Reste in den erfindungsgemäßen Verbindungen gegebenenfalls substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt.

Bevorzugt sind Verbindungen der allgemeinen Formel (I),
in welcher
- R¹: für 1-Aza-bicyclo[2.2.2]oct-3-yl steht,
und R² und R³ die oben angegebene Bedeutung haben.

Ebenfalls bevorzugt sind Verbindungen der allgemeinen Formel (I),
in welcher
- R²: für 9- bis 10-gliedriges Heteroaryl oder Naphthyl steht, wobei die Ringe gegebenenfalls durch 1 bis 3 Reste ausgewählt aus der Gruppe Wasserstoff, Halogen, Cyano, Trifluormethyl, Trifluormethoxy, Nitro, (C₁-C₄)-Alkyl, (C₁-C₄)-Alkoxy, (C₁-C₄)-Alkylthio substituiert sind,
und R¹ und R³ die oben angegebene Bedeutung haben.

Besonders bevorzugt sind Verbindungen der allgemeinen Formel (I),
in welcher
- R²: für Indolyl, Benzoimidazolyl, Benzotriazolyl, Benzothiophenyl, Benzofuranyl, Chinolinyl, Isochinolyl, Benzopyrazinyl, Benzopyrimidinyl, Benzopyridizanyl oder Naphthyl steht, wobei die Ringe gegebenenfalls durch 1 bis 3 Reste ausgewählt aus der Gruppe Wasserstoff, Halogen, Cyano, Trifluormethyl, Trifluormethoxy, Nitro, (C₁-C₄)-Alkyl, (C₁-C₄)-Alkoxy, (C₁-C₄)-Alkylthio substituiert sind,
und R¹ und R³ die oben angegebene Bedeutung haben.

Ganz besonders bevorzugt sind Verbindungen der allgemeinen Formel (I),
in welcher
- R²: für Benzotriazolyl, Benzothiophenyl, Chinolinyl, Benzopyrazinyl oder Naphthyl steht, wobei die Ringe gegebenenfalls durch 1 bis 3 Reste ausgewählt aus der Gruppe Wasserstoff, Halogen, Cyano, Trifluormethyl, Trifluormethoxy, Nitro, (C₁-C₄)-Alkyl, (C₁-C₄)-Alkoxy, (C₁-C₄)-Alkylthio substituiert sind,
und R¹ und R³ die oben angegebene Bedeutung haben.

Ebenfalls bevorzugt sind Verbindungen der allgemeinen Formel (I),
in welcher
- R³: für Wasserstoff oder Methyl steht,
und R¹ und R² die oben angegebene Bedeutung haben.

Besonders bevorzugt sind Verbindungen der allgemeinen Formel (I),
in welcher
- R³: für Wasserstoff steht,
und R¹ und R² die oben angegebene Bedeutung haben.

Ganz besonders bevorzugt sind Kombinationen von zwei oder mehreren der oben genannten Vorzugsbereiche.

Ebenfalls ganz besonders bevorzugt sind Verbindungen der allgemeinen Formel (I),
in welcher
- R¹: für 1-Aza-bicyclo[2.2.2]oct-3-yl steht,
- R²: für Benzotriazolyl, Benzothiophenyl, Chinolinyl, Benzopyrazinyl oder Naphthyl steht, wobei die Ringe gegebenenfalls durch 1 bis 3 Reste ausgewählt aus der Gruppe Wasserstoff, Halogen, Cyano, Trifluormethyl, Trifluormethoxy, Nitro, (C₁-C₄)-Alkyl, (C₁-C₄)-Alkoxy, (C₁-C₄)-Alkylthio substituiert sind,
und
- R³: für Wasserstoff steht.

Die Erfindung betrifft weiterhin Verfahren zur Herstellung der Verbindungen der Formel (I), dadurch gekennzeichnet, dass man
Verbindungen der allgemeinen Formel (II),

R¹-CO-X (II)

in welcher R¹ die oben genannte Bedeutung hat, und
- X: für Hydroxy oder eine geeignete Abgangsgruppe steht,
mit einer Verbindung der allgemeinen Formel (III),

HNR¹R² (III)

in welcher
R² und R³ die oben genannte Bedeutung haben,
in einem inerten Lösungsmittel gegebenenfalls in Gegenwart eines Kondensationsmittels und gegebenenfalls in Gegenwart einer Base umsetzt.

Wenn X eine Abgangsgruppe ist, sind Chloro, Mesyloxy und Isobutyloxycarbonyloxy, besonders Chloro bevorzugt.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, 1,2-Dimethoxyethan, Dimethylsulfoxid, Acetonitril oder Pyridin, bevorzugt sind Tetrahydrofuran, Dimethylformamid oder Chloroform.

Kondensationsmittel sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N',N'-tetramethyl-uroniumhexafluorophosphat (HATU) oder Benzotriazol-1-yloxytris(dimethylamino)-phosphoniumhexafluoro-phosphat (BOP), oder Mischungen aus diesen.

Gegebenenfalls kann es vorteilhaft sein, diese Kondensationsmittel in Gegenwart eines Hilfsnucleophils wie z.B. 1-Hydroxybenztriazol (HOBt) zu verwenden.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Besonders bevorzugt ist die Kombination von N-(3-Dimethylaminoisopropyl)-N'-ethylcarbodiimid-Hydrochlorid (EDC) und 1-Hydroxybenztriazol (HOBt) in Dimethylformamid.

Besonders bevorzugt ist die Kombination von N,N'-Diisopropylcarbodiimid (IDC) und Pentafluorophenol in DCM oder DMF. Der Einsatz von an Harz gebundenem Tetrafluorphenol und N,N'-Diisopropylcarbodiimid (IDC) ist ebenfalls besonders bevorzugt (Syntheseschema 1). Die Herstellung des an Harz gebundenen Tetrafluorophenols und dessen Einsatz erfolgt vorzugsweise gemäß Salvino et al. *J. Comb. Chem.* **2000,** *6,* 691-697.

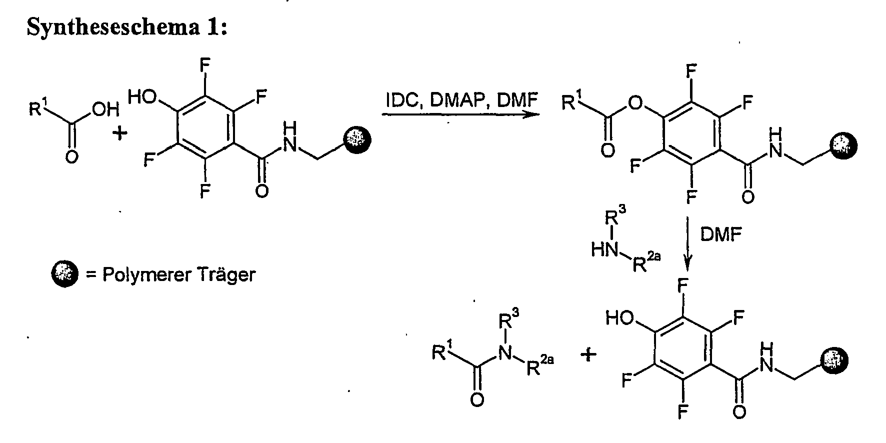

Vorzugsweise wird das erfindungsgemäße Verfahren in einem Temperaturbereich von Raumtemperatur bis 50°C bei Normaldruck durchgeführt.

Die Verbindungen der allgemeinen Formeln (II) und (III) sind bekannt oder lassen sich nach bekannten Verfahren aus den entsprechenden Edukten synthetisieren (vgl. z.B. Comprehensive Heterocyclic Chemistry', Katritzki et al., Hrsg.; Elsevier, 1996).

So können beispielsweise Verbindungen der Formel (II) gemäß literaturbekannten Verfahren hergestellt werden.

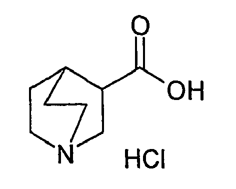

3-Chinuklidincarbonsäure Hydrochlorid: Orlek et al. *J. Med. Chem.* **1991**, *34,* 2726.

Verbindungen der Formel (III) sind aus den entsprechenden Carbonsäuren durch dem Fachmann bekannte Reaktionen zugänglich. So lassen sich Aniline durch z. B. Curtius- oder Hoffmann-Abbau aus Carbonsäuren oder deren Derivaten herstellen (vgl. z.B. 'Organikum', Wiley-VCH; 1999). Besonders geeignet ist der Einsatz von Diphenylphosphorazidat (DPPA) zur Generierung des als Zwischenstufe auftretenden Isocyanates, welches anschließend mit Wasser zur Zielverbindung raegiert. (T. Shioiri and S. Yamada, *Chem. Pharm. Bull.* **1974,** *22*, 859; Shioiri et al. *J. Am. Chem. Soc* **1972,** *94,* 6203.

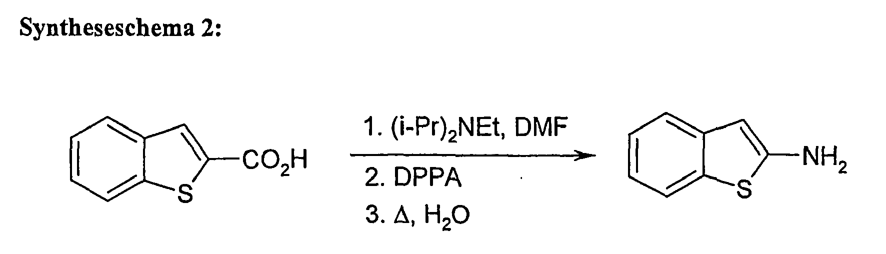

Die erfindungsgemäßen Verbindungen der allgemeinen Formel (I) eignen sich zur Verwendung als Arzneimittel zur Behandlung und/oder Prophylaxe von Krankheiten bei Menschen und/oder Tieren.

Die erfindungsgemäßen Verbindungen zeigen ein nicht vorhersehbares, wertvolles pharmakologisches Wirkspektrum.

Sie zeichnen sich als Liganden, insbesondere Agonisten am α7-nAChR aus.

Die erfindungsgemäßen Verbindungen können aufgrund ihrer pharmakologischen Eigenschaften allein oder in Kombination mit anderen Arzneimitteln zur Behandlung und/oder Prävention von kognitiven Störungen , insbesondere der Alzheimerschen Krankheit eingesetzt werden. Wegen ihrer selektiven Wirkung als α7-nAChR Agonisten eignen sich die erfindungsgemäßen Verbindungen besonders zur Verbesserung der Wahrnehmung, Konzentrationsleistung, Lernleistung, oder Gedächtnisleistung insbesondere nach kognitiven Störungen, wie sie beispielsweise bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatisches Schädel Hirn Trauma, allgemeine Konzentrationsstörungen, Konzentrationsstörungen in Kindern mit Lern- und Gedächtnisproblemen, Attention Deficit Hyperactivity Disorder, Alzheimersche Krankheit, Vaskuläre Demenz, Demenz mit Lewy-Körperchen, Demenz mit Degeneration der Frontallappen einschließlich des Pick's Syndroms, Parkinsonsche Krankheit, Progressive nuclear palsy, Demenz mit corticobasaler Degeneration, Amyotrophe Lateralsklerose (ALS), Huntingtonsche Krankheit, Multiple Sklerose, Thalamische Degeneration, Creutzfeld-Jacob-Demenz, HIV-Demenz, Schizophrenie, Schizophrenie mit Demenz oder Korsakoff-Psychose.

Die erfindungsgemäßen Verbindungen können allein oder in Kombination mit anderen Arzneimitteln eingesetzt werden zur Prophylaxe und Behandlung von akuten und/oder chronischen Schmerzen (für eine Klassifizierung siehe "Classification of Chronic Pain, Descriptions of Chronic Pain Syndromes and Definitions of Pain Terms", 2. Aufl., Meskey und Begduk, Hrsg.; IASP-Press, Seattle, 1994), insbesondere zur Behandlung von Krebs-induzierten Schmerzen und chronischen neuropathischen Schmerzen, wie zum Beispiel, bei diabetischer Neuropathie, postherpetischer Neuralgie, peripheren Nervenbeschädigungen, zentralem Schmerz (beispielsweise als Folge von cerebraler Ischämie) und trigeminaler Neuralgie, und anderen chronischen Schmerzen, wie zum Beispiel Lumbago, Rückenschmerz (low back pain) oder rheumatischen Schmerzen. Daneben eignen sich diese Substanzen auch zur Therapie von primär akuten Schmerzen jeglicher Genese und von daraus resultierenden sekundären Schmerzzuständen, sowie zur Therapie chronifizierter, ehemals akuter Schmerzzustände.

Die *in vitro*-Wirkung der erfindungsgemäßen Verbindungen kann in folgenden Assays gezeigt werden:

### 1. Bestimmung der Affinität von Testsubstanzen für α7-nAChR durch Inhibition von [³H]Methyllycaconitine-Bindung an Rattenhirnmembranen

Der [³H]-Methyllycaconitine Bindungstest ist eine Modifikation der von Davies et al. (*Neuropharmacol.* **1999,** *38,* 679-690) beschriebenen Methode.

Rattenhimgewebe (Hippocampus oder Gesamthirn) wird in Homogenisierungspuffer (10 % w/v) (0.32 M Sucrose, 1 mM EDTA, 0.1 mM Phenylmethylsulfonyl fluorid (PMSF), 0,01 % (w/v) NaN₃, pH 7,4, 4 °C) bei 600 rpm in einem Glashomogenisator homogenisiert. Das Homogenat wird zentrifugiert (1000 x g, 4°C, 10 min) und der Überstand wird abgenommen. Das Pellet wird erneut suspendiert (20 % w/v) und zentrifugiert (1000 x g, 4°C, 10 min). Die beiden Überstände werden vereinigt und zentrifugiert (15.000 x g, 4°C, 30 min). Dieses Pellet wird als P2 Fraktion bezeichnet.

Das P2-Pellet wird zweimal mit Bindungspuffer gewaschen (50 mM Tris-HCl, 1 mM MgCl₂, 120 mM NaCl, 5 mM KCl, 2 mM CaCl₂, pH 7.4) und zentrifugiert (15.000 x g, 4°C, 30min).

Die P2 Membranen werden in Bindungspuffer resuspendiert und in einem Volumen von 250 µl (Membranproteinmenge 0,1 - 0,5 mg) für 2,5 h bei 21°C inkubiert in der Gegenwart von 1-5 nM [³H]-Methyllycaconitine, 0.1 % (w/v) BSA (bovines Serumalbumin) und verschiedenen Konzentrationen der Testsubstanz. Die unspezifische Bindung wird bestimmt durch Inkubation in der Gegenwart von 1 µM α-Bungarotoxin oder 100 µM nicotine oder 10 µM MLA (Methyllycaconitine).

Die Inkubation wird beendet durch Zugabe von 4 ml PBS (20 mM Na₂HPO₄, 5 mM KH₂PO₄, 150 mM NaCl, pH 7,4, 4°C) und Filtration durch Typ A/E glass fibre filters (Gelman Sciences), die vorher 3 h in 0,3 % (v/v) Polyethyleneimine (PEI) eingelegt waren. Die Filter werden zweimal mit 4 ml PBS (4°C) gewaschen und die gebundene Radioaktivität durch Szintillationsmessung bestimmt. Alle Tests werden in Dreifachbestimmungen durchgeführt. Aus dem IC₅₀-Wert der Verbindungen (Konzentration der Testsubstanz, bei der 50 % des am Rezeptor gebundenen Liganden verdrängt werden), der Dissoziationskonstante K_{D} und der Konzentration L von [³H]Methyllycaconitine wurde die Dissoziationskonstante der Testsubstanz Kᵢ bestimmt (Kᵢ = IC₅₀/(1+L/K_{D})),

Anstelle von [³H]-Methyllycaconitine können auch andere α7-nAChR-selektive Radioliganden wie z.B. [¹²⁵I]-α-Bungarotoxin oder unselektive nAChR-Radioliganden gemeinsam mit Inhibitoren anderer nAChR eingesetzt werden.

Die Eignung der erfindungsgemäßen Verbindungen zur Behandlung von kognitiven Störungen kann in folgenden Tiermodellen gezeigt werden:

### 2. Objekt-Wiedererkennungstest

Der Objekt-Wiedererkennungstest ist ein Gedächtnistest. Er misst die Fähigkeit von Ratten (und Mäusen), zwischen bekannten und unbekannten Objekten zu unterscheiden.

Der Test wird wie beschrieben durchgeführt (Blokland et al. *NeuroReport* **1998,** *9,* 4205-4208; Ennaceur, A., Delacour, J.,. *Behav. Brain Res.* **1988,** *31,* 47-59; Ennaceur, A., Meliani, K.,. *Psychopharmacology* **1992,** *109,* 321-330; Prickaerts, et al. *Eur. J. Pharmacol.* **1997,** *337,* 125-136).

In einem ersten Durchgang wird eine Ratte in einer ansonsten leeren größeren Beobachtungsarena mit zwei identischen Objekten konfrontiert. Die Ratte wird beide Objekte ausgiebig untersuchen, d.h. beschnüffeln und berühren. In einem zweiten Durchgang, nach einer Wartezeit von 24 Stunden, wird die Ratte erneut in die Beobachtrungsarena gesetzt. Nun ist eines der bekannten Objekte durch ein neues, unbekanntes Objekt ersetzt. Wenn eine Ratte das bekannte Objekt wiedererkennt, wird sie vor allem das unbekannte Objekt untersuchen. Nach 24 Stunden hat eine Ratte jedoch normalerweise vergessen, welches Objekt sie bereits im ersten Durchgang untersucht hat, und wird daher beide Objekte gleichstark inspektieren. Die Gabe einer Substanz mit lern-und gedächtnisverbessernder Wirkung wird dazu führen, dass eine Ratte das bereits 24 Stunden vorher, im ersten Durchgang, gesehene Objekt als bekannt wiedererkennt. Sie wird das neue, unbekannte Objekt ausführlicher untersuchen als das bereits bekannte. Diese Gedächtnisleistung wird in einem Diskriminationsindex ausgedrückt. Ein Diskiminationsindex von Null bedeutet, dass die Ratte beide Objekte, das alte und das neue, gleichlang untersucht; d.h. sie hat das alte Objekt nicht wiedererkannt und reagiert auf beide Objekte als wären sie unbekannt und neu. Ein Diskriminationsindex größer Null bedeutet, dass die Ratte das neue Objekt länger inspektiert als das alte; d.h. die Ratte hat das alte Objekt wiedererkannt.

### 3. Sozialer Wiedererkennungstest:

Der Soziale Wiedérerkennungstest ist ein Test zur Prüfung der lern- oder gedächtnisverbessernden Wirkung von Testsubstanzen.

Erwachsene Ratten, die in Gruppen gehalten werden, werden 30 Minuten vor Testbeginn einzeln in Testkäfige gesetzt. Vier Minuten vor Testbeginn wird das Testtier in eine Beobachtungsbox gebracht. Nach dieser Adaptationszeit wird ein juveniles Tier zu dem Testtier gesetzt und 2 Minuten lang die totale Zeit gemessen, die das adulte Tier das Junge investigiert (Trial 1). Gemessen werden alle deutlich auf das Jungtier gerichteten Verhaltensweisen, d.h. ano-genitale Inspektion, Verfolgen sowie Fellpflege, bei denen das Alttier einen Abstand von höchstens 1 cm zu dem Jungtier hat. Danach wird das Juvenile herausgenommen und das Adulte in seinem Testkäfig belassen (bei 24 Stunden Retention wird das Tier in seinen Heimkäfig zurückgesetzt). Vor oder nach dem ersten Test wird das Versuchstier mit Substanz behandelt. Je nach Zeitpunkt der Substanzgabe wird das Erlernen oder das Speichern der Information über das Jungtier durch die Substanz beeinflusst. Nach einem festgelegten Zeitraum (Retention) wird der Test wiederholt (Trial 2). Je größer die Differenz zwischen den in Trial 1 und 2 ermittelten Investigationszeiten, desto besser hat sich das adulte Tier an das Jungtier erinnert

Die erfindungsgemäßen Verbindungen der allgemeinen Formel (I) eignen sich zur Verwendung als Arzneimittel für Menschen und Tiere.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Verbindungen der Formel (I) bestehen, sowie Verfahren zur Herstellung dieser Zubereitungen.

Die Verbindungen der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Verbindungen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, insbesondere perlingual oder intravenös. Sie kann aber auch durch Inhalation über Mund oder Nase, beispielsweise mit Hilfe eines Sprays erfolgen, oder topisch über die Haut.

Im Allgemeinen hat es sich als vorteilhaft erwiesen, Mengen von etwa 0,001 bis 10 mg/kg, bei oraler Anwendung vorzugsweise etwa 0,005 bis 3 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Abkürzungen:

- DMF: *N,N-*Dimethylformamid
- DMSO: Dimethylsulfoxid
- HOBt: 1-Hydroxy-1H-benzotriazol x H₂O
- NMR: Kernresonanzspektroskopie
- RT: Raumtemperatur
- TBTU: *O*-(Benzotriazol-1-yl)-*N,N,N',N'*-tetramethyluronium-Tetrafluoroborat
- THF: Tetrahydrofuran

### LC-MS Methode:

Gerätetyp MS: Micromass Platform LCZ
Ionisierung: ESI positiv
Gerätetyp HPLC: HP 1100
UV-Detektor DAD: 208-400 nm
Ofentemp.: 40°C
Säule: Symmetry C 18
50 mm x 2.1 mm; 3.5 µm
Gradient:

| Zeit (min) | A: % | B: % | Fluß |
|---|---|---|---|
| | | | (ml/min) |
| 0.00 | 10.0 | 90.0 | 0.50 |
| 4.00 | 90.0 | 10.0 | 0.50 |
| 6.00 | 90.0 | 10.0 | 0.50 |
| 6.10 | 10.0 | 90.0 | 1.00 |
| 7.50 | 10.0 | 90.0 | 0.50 |

A: Acetonitril + 0.1% Ameisensäure
B: Wasser + 0.1% Ameisensäure

### Ausgangsverbindungen:

### Beispiel 1 A

### 3-Chinuklidincarbonsäurechlorid Hydrochlorid

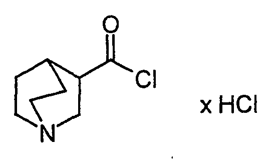

3-Chinuklidincarbonsäure Hydrochlorid wurde gemäß Orlek et al. *J. Med. Chem.* **1991**, 34, 2726 hergestellt.

500 mg (2,61 mmol) 3-Chinuklidincarbonsäure werden zusammen mit 1,9 ml (26,09 mmol) Thionylchorid 2 h unter Rückfluss erhitzt. Das Reaktionsgemisch wird unter reduziertem Druck vom überschüssigen Thionylchlorid befreit. Es wird zweimal mit je 20 ml Toluol versetzt und jeweils bis zur Trockene eingeengt. Das so erhaltene Produkt wird direkt ohne weitere Reinigung umgesetzt.

### Beispiel 2 A

### 7-Brom-1-benzothiophen-2-amin

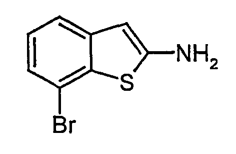

156 mg (0,61 mmol) 7-Brom-1-benzothiophen-2-carbonsäure werden zusammen mit 156,8 mg (1.21 mmol) N,N-Diisopropylethylamin in 4,0 ml DMF vorgelegt. Bei 0°C werden 183,7 mg (0.67 mmol) Diphenylphosphorazidat zugegeben. Das Reaktionsgemisch wird über Nacht bei 8°C stehengelassen und dann in Eiswasser eingerührt. Es wird mit Essigsäure neutralisiert und der entstandene Niederschlag abgesaugt und vorsichtig bei 40°C getrocknet. Anschließend wird der Feststoff in Xylol suspendiert und 1 h unter Rückfluß erhitzt. Das Solvens wird unter reduziertem Druck entfernt und der Rückstand in Wasser gegeben und 3 h lang erhitzt. Die wässrige Phase wird mehrmals mit Ethylacetat extrahiert. Die organische Phase wird über Natriumsulfat getrocknet, anschließend wird das Lösungsmittel unter reduziertem Druck entfernt. Es werden 128 mg der Titelverbindung in einer Reinheit isoliert, die für weitere Umsetzungen geeignet ist. Das Amin wird ohne weitere Aufreinigung weiter umgesetzt.
LC-MS: Rₜ = 5.25 min., MS (ESIpos): *m*/*z* = 229 (M+H)⁺.

### Ausführungsbeispiele:

### Beispiel 1

### N-(2-Naphthyl)chinuklidin-3-carboxamid-Hydrochlorid

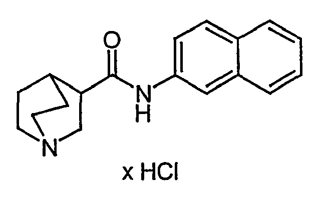

Zu einer Lösung von 540 mg (2.57 mmol) 3-Chinuklidincarbonsäurechlorid-Hydrochlorid und Pyridin (620 mg, 4,8 mmol) in 3 ml Dimethylformamid werden bei 0°C 740 mg (5,14 mmol) 2-Naphthylamin gegeben. Das Gemisch wird über Nacht bei RT gerührt. Zur Aufarbeitung wird eingeengt und in Dichlormethan aufgenommen. Das Rohprodukt wird über Kieselgel chromatographiert (Laufmittel: Dichlormethan / Methanol 10:1, 5:1). Das erhaltene Produkt kristallisiert aus Dichlormethan aus, wird abgesaugt und getrocknet.
Ausbeute: 26 % d.Th. des Hydrochlorids
¹H-NMR (200,1 MHz, DMSO-d₆): δ = 10,60 (br. s, 1H), 10,10 (br. s, 1H), 8,35 (d, 1H), 7,95-7,75 (m, 3H), 7,65 (dd, 1H), 7,60-7,45 (m, 2H), 3,65 (m, 1H), 3,45-,10 (m, 7H), 2,05-1,85 (m, 2H), 1,85-1,70 (m, 2H)
MS (ESIpos): *m*/*z* = 281 (M+H)⁺ (freie Base)
LC-MS: Rₜ = 2,65 min., MS (ESIpos): *m*/*z* = 281 (M+H)⁺ (freie Base).

### Beispiel 2

### N-(6-Chinolinyl)chinuklidin-3-carboxamid-Hydrochlorid

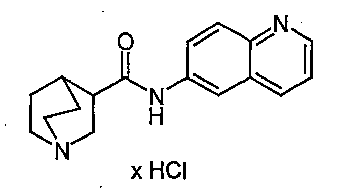

172 mg (1,19 mmol) 6-Aminochinolin werden unter Argon in 3 ml DMF zusammen mit 461 mg (3,57 mmol) N,N-Diisopropylethylamin bei 0°C vorgelegt. 250 mg (2,57 mmol) 3-Chinuklidincarbonsäurechlorid-Hydrochlorid werden in wenig trockenem DMF gelöst und bei 0°C in das Reaktionsgemisch getropft. Es wird über Nacht bei Raumtemperatur nachgerührt. Zur Aufarbeitung wird eingeengt und in Dichlormethan aufgenommen. Das Rohprodukt wird über Kieselgel 60 chromato-graphiert (Laufmittel: Dichlormethan / Ethylacetat 10:1, dann Dichlormethan / Methanol 10:1, 3:1). Die Produkt-Fraktionen werden vereinigt und mittels präparativer HPLC (RP18-Säule; Laufmittel: Acetonitril / Wasser, Gradient 1:99 → 30:70) feingereinigt. Ausbeute: 28 mg (7 % d.Th.) des Hydrochlorids
¹H-NMR (200,1 MHz, DMSO-d₆): δ = 11,00 (br. s, 1H), 10,19 (br. s, 1H), 9,06-9,04 (m, 1H), 8,88-8,85 (m, 1H), 8,70-8,68 (m, 1H), 8,27-8,23 (m, 1H), 8,12-8,06 (m, 1H), 7,89-7,83 (m, 1H), 3,70-3,50 (m, 1H), 3,40-3,10 (m, 7H), 2,00-1,80 (m, 2H), 1,80-1,65 (m, 2H)
MS (ESIpos): *m*/*z* = 282 (M+H)⁺ (freie Base)
LC-MS: Rₜ =0.30 min., MS (ESIpos): *m*/*z* = 282 (M+H)⁺ (freie Base).

### Beispiel 3

### N-(3a,7a-Dihydro-1H-indazol-6-yl)chinuklidin-3-carboxamid-Hydrochlorid

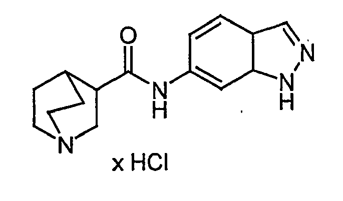

Analog der in Beispiel 2 beschriebenen Vorschrift werden 250 mg (2.57 mmol) 3-Chinuklidincarbonsäurechlorid, 461 mg (3,57 mmol) N,N-Diisopropylethylamin, katalytische Mengen DMAP (ca. 1 mg) und 158 mg (1,19 mmol) 4-Aminoindazol umgesetzt.
Ausbeute: 55 mg (15 % d.Th.) des Hydrochlorids
¹H-NMR (200,1 MHz, DMSO-d₆): δ = 10,55 (br. s, 1H), 10,05 (br. s, 1H), 8,15 (s, 1H), 7,97 (d, 1H), 7,67 (d, 1H), 7,14 (dd, 1H), 3,70-3,60 (m, 1H), 3,40-3,15 (m, 7H), 2,00-1,85 (m, 2H), 1,80-1,65 (m, 2H)
MS (ESIpos): *m*/*z* = 271 (M+H)⁺ (freie Base).

### Beispiel 4

### N-(6-Chinoxalinyl)chinuklidin-3-carboxamid-Hydrochlorid

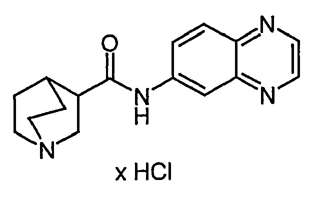

Analog der in Beispiel 2 beschriebenen Vorschrift werden 250 mg (2,57 mmol) 3-Chinuklidincarbonsäurechlorid, 461 mg (3,57 mmol) N,N-Diisopropylethylamin, katalytische Mengen DMAP (ca. 1 mg) und 173 mg (1,19 mmol) 6-Chinoxalinylamin umgesetzt.
Ausbeute: 58 mg (15 % d.Th.) des Hydrochlorids
¹H-NMR (200,1 MHz, DMSO-d₆): δ = 10,90 (s, 1H), 9,99 (br. s, 1H), 8,90-8,88 (m, 2H), 8,84-8,83 (m, 2H), 8,10-7,94 (m, 2H), 3,60-3,50 (m, 1H), 3,30-3,05 (m, 7H), 2,00-1,80 (m, 2H), 1,80-1,65 (m, 2H)
MS (ESIpos): *m*/*z* = 283 (M+H)⁺ (freie Base).

## Patentansprüche

1. Verbindungen der allgemeinen Formel (I), 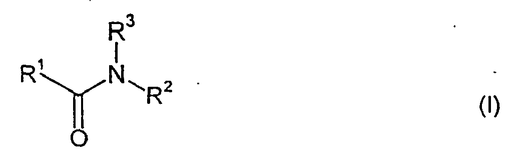 in welcher
R¹ für 1-Aza-bicyclo[3.2.1]octyl, 1-Aza-bicyclo[3.3.1]nonyl oder 1-Aza-bicyclo[2.2.2]oct-3-yl steht,
wobei der Bicycloalkylrest gegebenenfalls durch (C₁-C₆)-Alkyl substituiert ist,
R² für 8- bis 10-gliedriges Heteroaryl, Naphthyl oder Azulenyl steht, wobei die Ringe gegebenenfalls durch Reste ausgewählt aus der Gruppe Wasserstoff, Halogen, Formyl, Carbamoyl, Cyano, Trifluormethyl, Trifluormethoxy, Nitro, (C₁-C₆)-Alkyl, (C₁-C₆)-Alkoxy, (C₁-C₆)-Alkylthio substituiert sind,
und
R³ für Wasserstoff oder (C₁-C₆)-Alkyl steht,
und deren Salze, Solvate und Solvate der Salze.

2. Verbindungen nach Anspruch 1, wobei
R¹ für 1-Aza-bicyclo[2.2.2]oct-3-yl steht,
und R² und R³ die in Anspruch 1 angegebene Bedeutung haben.

3. Verbindungen nach Anspruch 1 oder 2, wobei
R¹ für 1-Aza-bicyclo[2.2.2]oct-3-yl steht,
R² für Benzotriazolyl, Benzothiophenyl, Chinolinyl, Benzopyrazinyl oder Naphthyl steht, wobei die Ringe gegebenenfalls durch 1 bis 3 Reste ausgewählt aus der Gruppe Wasserstoff, Halogen, Cyano, Trifluormethyl, Trifluormethoxy, Nitro, (C₁-C₄)-Alkyl, (C₁-C₄)-Alkoxy, (C₁-C₄)-Alkylthio substituiert sind,
und
R³ für Wasserstoff steht.

4. Verfahren zur Herstellung von Verbindungen der allgemeinen Formel (I) nach Anspruch 1, **dadurch gekennzeichnet, dass** man Verbindungen der allgemeinen Formel (II),
R¹-CO-X (II)
in welcher
R¹ die oben genannte Bedeutung hat, und
X für Hydroxy oder eine geeignete Abgangsgruppe steht,
mit einer Verbindung der allgemeinen Formel (III)
HNR²R³ (III)
in welcher
R² und R³ die oben genannte Bedeutung haben, und
in einem inerten Lösungsmittel gegebenenfalls in Gegenwart eines Kondensationsmittels und gegebenenfalls in Gegenwart einer Base umsetzt.

5. Verbindungen nach einem der Ansprüche 1 bis 3 zur Behandlung und/oder Prophylaxe von Krankheiten.

6. Arzneimittel enthaltend mindestens eine der Verbindungen nach einem der Ansprüche 1 bis 3 in Zusammenmischung mit mindestens einem pharmazeutisch verträglichen, im wesentlichen nichtgiftigen Träger oder Exzipienten.

7. Verwendung von Verbindungen nach einem der Ansprüche 1 bis 3 zur Herstellung eines Arzneimittels zur Verbesserung der Wahrnehmung, Konzentrationsleistung, Lernleistung und/oder Gedächtnisleistung.

8. Verwendung von Verbindungen nach einem der Ansprüche 1 bis 3 zur Herstellung eines Arzneimittels zur Behandlung und/oder Prophylaxe von Störungen der Wahrnehmung, Konzentrationsleistung, Lernleistung und/oder Gedächtnisleistung.

9. Arzneimittel nach Anspruch 6 zur Behandlung und/oder Prophylaxe von Störungen der Wahrnehmung, Konzentrationsleistung, Lernleistung und/oder Gedächtnisleistung.

## Claims

1. Compounds of the general formula (I) 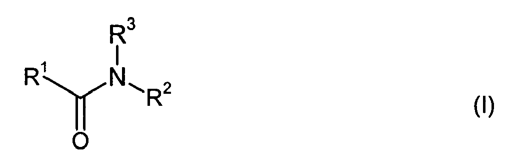 in which
R¹ is 1-azabicyclo[3.2.1]octyl, 1-azabicyclo[3.3.1]nonyl or 1-azabicyclo[2.2.2]oct-3-yl, where the bicycloalkyl radical is optionally substituted by (C₁-C₆)-alkyl,
R² is 8- to 10-membered heteroaryl, naphthyl or azulenyl, where the rings are optionally substituted by radicals selected from the group of hydrogen, halogen, formyl, carbamoyl, cyano, trifluoromethyl, trifluoromethoxy, nitro, (C₁-C₆)-alkyl, (C₁-C₆)-alkoxy, (C₁-C₆)-alkylthio,
and
R³ is hydrogen or (C₁-C₆)-alkyl,
and their salts, solvates and solvates of the salts.

2. Compounds according to Claim 1, where
R¹ is 1-azabicyclo[2.2.2]oct-3-yl,
and R² and R³ have the meaning indicated in claim 1.

3. Compounds according to Claim 1 or 2, where
R¹ is 1-azabicyclo[2.2.2]oct-3-yl,
R² is benzotriazolyl, benzothiophenyl, quinolinyl, benzopyrazinyl or naphthyl, where the rings are optionally substituted by 1 to 3 radicals selected from the group of hydrogen, halogen, cyano, trifluoromethyl, trifluoromethoxy, nitro, (C₁-C₄)-alkyl, (C₁-C₄)-alkoxy, (C₁-C₄)-alkylthio,
and
R³ is hydrogen.

4. Process for preparing compounds of the general formula (I) according to Claim 1, **characterized in that** compounds of the general formula (II)
R¹-CO-X (II)
in which
R¹ has the abovementioned meaning, and
X is hydroxyl or a suitable leaving group,
are reacted with a compound of the general formula (III)
HNR¹R² (III)
in which
R² and R³ have the abovementioned meaning,
in an inert solvent, where appropriate in the presence of a condensing agent and where appropriate in the presence of a base.

5. Compounds according to any one of Claims 1 to 3 for the treatment and/or prophylaxis of diseases.

6. Medicament comprising at least one compound according to any of Claims 1 to 3 mixed with at least one pharmaceutically acceptable, essentially nontoxic carrier or excipient.

7. Use of compounds according to any of Claims 1 to 3 for producing a composition for improving perception, concentration, learning and/or memory.

8. Use of compounds according to any of Claims 1 to 3 for producing a medicament for the treatment and/or prophylaxis of impairments of perception, concentration, learning and/or memory.

9. Medicament according to Claim 6 for the treatment and/or prophylaxis of impairments of perception, concentration, learning and/or memory.

## Revendications

1. Composés de formule générale (I) : 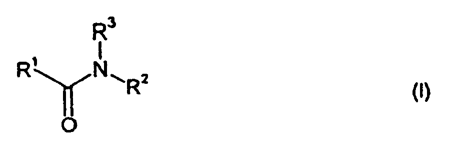 dans laquelle
R¹ représente un reste 1-aza-bicyclo[3.2.1]octyle, 1-aza-bicyclo[3.3.1]nonyle ou 1-aza-bicyclo[2.2.2]oct-3-yle, le reste bicycloalkyle étant éventuellement substitué par un radical alkyle en C₁ à C₆,
R² représente un reste hétéroaryle octogonal à décagonal, naphtyle ou azulényle, le noyau étant éventuellement substitué par des restes choisis dans le groupe hydrogène, halogène, formyle, carbamoyle, cyano, trifluorométhyle, trifluorométhoxy, nitro, alkyle en C₁ à C₆, alkoxy en C₁ à C₆, alkylthio en C₁ à C₆,
et
R³ représente l'hydrogène ou un reste alkyle en C₁ à C₆,
et leurs sels, leurs produits de solvatation et les produits de solvatation des sels.

2. Composés suivant la revendication 1, dans lesquels
R¹ est un reste 1-aza-bicyclo[2.2.2]oct-3-yle,
et R² et R³ ont la définition indiquée dans la revendication 1.

3. Composés suivant la revendication 1 ou 2, dans lesquels
R¹ est un reste 1-aza-bicyclo[2.2.2]oct-3-yle,
R² est un reste benzotriazolyle, benzothiophényle, quinolinyle, benzopyrazinyle ou naphtyle, le noyau étant éventuellement substitué par 1 à 3 restes choisis dans le groupe hydrogène, halogène, cyano, trifluorométhyle, trifluorométhoxy, nitro, alkyle en C₁ à C₄, alkoxy en C₁ à C₄, alkylthio en C₁ à C₄,
et
R³ représente l'hydrogène.

4. Procédé de production de composés de formule générale (I) suivant la revendication 1, **caractérisé en ce qu'**on fait réagir des composés de formule générale (II) :
**R**^{**1**}**-CO-X** **(II)**
dans laquelle
R¹ a la définition mentionnée ci-dessus,
X est un groupe hydroxy ou un groupe partant convenable, avec un composé de formule générale (III)
**HNR**^{**2**}**R**^{**3**} **(III)**
dans laquelle
R² et R³ ont la définition mentionnée ci-dessus, et
dans un solvant inerte, éventuellement en présence d'un agent de condensation et, le cas échéant, en présence d'une base.

5. Composés suivant l'une des revendications 1 à 3, destinés au traitement et/ou à la prophylaxie de maladies.

6. Médicament contenant au moins l'un des composés suivant l'une des revendications 1 à 3, en mélange avec au moins un support ou excipient pharmaceutiquement acceptable, essentiellement non toxique.

7. Utilisation de composés suivant l'une des revendications 1 à 3, pour la préparation d'un médicament destiné à améliorer la perception, la capacité de concentration, la capacité d'apprentissage et/ou la capacité de mémoire.

8. Utilisation de composés suivant l'une des revendications 1 à 3, pour la préparation d'un médicament destiné au traitement et/ou à la prophylaxie de troubles de la perception, de la capacité de concentration, de la capacité d'apprentissage et/ou de la capacité de mémoire.

9. Médicament suivant la revendication 6, destiné au traitement et/ou à la prophylaxie de troubles de la perception, de la capacité de concentration, de la capacité d'apprentissage et/ou de la capacité de mémoire.
